# EUROPEAN PATENT APPLICATION

(11) **EP 4 621 048 A2**
(43) Date of publication of application: **24.09.2025**
(21) Application number: 25160600.0
(22) Date of filing: 27.02.2025
(51) Int. Cl.: C12N 5/079, G01N 33/68

(54) **METHOD FOR IDENTIFYING ASTROCYTE POPULATION HAVING NERVE- SUPPORTING FUNCTION OR AN ENHANCED FUNCTION**

(30) Priority: 21.03.2024 JP 2024044604
(71) Applicant: Ricoh Company, Ltd., Tokyo 143-8555 (JP)
(72) Inventor: IJICHI, Rie, Tokyo, 143-8555 (JP); SHIOYA, Yuina, Tokyo, 143-8555 (JP); HOSOYA, Toshihiko, Tokyo, 143-8555 (JP)
(74) Representative: Marks & Clerk LLP

(57) **Abstract**

A problem to be addressed by the present invention is to develop a nerve-supporting function marker of an astrocyte, and to develop, using the marker, a method for determining an increase in the nerve-supporting function of an astrocyte population cultured *in vitro.* Using a GFAP as an astrocyte nerve-supporting function marker, an astrocyte population having a high GFAP positive rate in the astrocyte population is identified as an astrocyte population having an enhanced nerve-supporting function.

## Description

### BACKGROUND OF THE INVENTION

### Field of the Invention

The present invention relates to a method for identifying an astrocyte population having a nerve-supporting function or an enhanced function.

### Description of the Related Art

An astrocyte is one of the gliacytes distributed in the brain, and has various functions such as the uptake of a neurotransmitter, the maintenance of the ionic environment around synapses, the control of the blood-brain barrier, energy supply to a neuron, and metabolism. In recent years, an astrocyte has been newly revealed as having a function for supporting neurons, and has been attracting attention as a cell that conditions the intracerebral environment (Non-Patent Documents 1 and 2). On the basis of this function, a technology has been developed, with which technology an astrocyte is cocultured with a neuron *in vitro* to make the neural activities more active (Non-Patent Document 3).

A marker for identifying and detecting an astrocyte is, for example, the following: a GFAP (glial fibrillary acidic protein) that is a main component protein in the cytoskeleton of an astrocyte; S100β that is a calcium-binding protein; or a catalyst enzyme ALDH1L1 (aldehyde dehydrogenase 1 family member L1) that converts NADP⁺ into NADPH (Non-Patent Document 4). In addition, a protein marker (function marker) that presents each function in an astrocyte is also known. Examples comprise a glucose transporter (GLUT1) protein that contributes to the intracellular ingestion of glucose in blood, an excitatory amino acid transporter/glutamate-aspartate transporter (EAAT1/GLAST) that is involved in the reuptake of a glutamate, and a glutamine synthetase that catalyzes a reaction that synthesizes glutamine from glutamic acid and ammonia (Non-Patent Document 4).

However, there has hitherto been no known marker capable of detecting an astrocyte having a nerve-supporting function. For example, a transcription factor NF1A (nuclear factor I-A) is known as a protein having a synapse-supporting function (Non-Patent Document 4). As disclosed (Non-Patent Document 5), the actual function of the transcription factor is involved in the expression of a gene for repairing a neuron when the neuron is damaged, and the function is not a function of the neuron, i.e., does not support neural activities. NF1A is expressed widely in many kinds of cells, and not practicably utilized as a nerve-supporting function marker of an astrocyte.

### SUMMARY OF THE INVENTION

A problem to be solved by the present invention is to develop and provide a marker of an astrocyte having the activity of a nerve-supporting function *in vitro,* and a method for determining, using the marker, whether an astrocyte population cultured *in vitro* is a cell population having a nerve-supporting function or an enhanced function.

To solve the above-described problem, the present inventors have vigorously made studies, and consequently get new knowledge that, when an astrocyte is cultured with a neuron *in vitro,* an astrocyte that highly expresses a GFAP achieves a nerve-supporting function strongly, compared with an astrocyte that hardly expresses a GFAP. As described above, a GFAP is known as a most common marker for identifying an astrocyte. However, a GFAP is expressed in not all the astrocytes. It has been revealed that there are many astrocytes in which no GFAP is expressed (Batiuk MY, et al., Nat Commun., 11 (1), 1220). There are also reports that, *in vivo*, a GFAP is highly expressed in an astrocyte increased in reactivity by the enhancement of a calcium signal (Li L., et al., Front. Cell. Neurosci., 2022, 16; https: //doi.org/10.3389/fncel.2022.850866), and that, as a result, the exchange of a substance between an astrocyte and a neuron is activated (Shigetomi E., et al., Int. J. Mol. Sci., 2019, 20 (4): 996; https://www.mdpi.com/1422-0067/20/4/996). However, an experiment by the present inventors has revealed that even an astrocyte that highly expresses a GFAP *in vitro* does not enable a calcium signal to be detected. From these results, the present inventors have found that a GFAP serves as a hitherto unknown *in vitro* marker for the nerve-supporting function of an astrocyte.

The present invention is based on the above-described new knowledge, and provides the following a nerve-supporting function marker of an astrocyte, the marker consisting of a GFAP, and a method including detecting a GFAP in an astrocyte population cultured *in vitro,* and, on the basis of the positive rate of the astrocyte population expressing the GFAP in the astrocyte population, determining whether the astrocyte population is an astrocyte population having a nerve-supporting function or an enhanced nerve-supporting function.

An astrocyte nerve-supporting function marker according to the present invention can provide a hitherto unknown marker for the nerve supporting function in an astrocyte *in vitro.*

According to an identifying method of the present invention , an astrocyte population having a nerve-supporting function or an enhanced function can be identified in an astrocyte population cultured *in vitro.*

### BRIEF DESCRIPTION OF THE DRAWINGS

FIG. 1 shows that a GFAP in an astrocyte derived from an iPS cell (iPSC) serves as a nerve-supporting function marker. FIGs. 1A1 to 1A4 illustrate iPS-cell-derived astrocytes not induced to express a GFAP, and FIGs. 1B1 to 1B4 illustrate iPS cell-derived astrocytes induced to express a GFAP.
FIG. 2 shows that a GFAP in an astrocyte as a primary cultured cell serves as a nerve-supporting function marker. FIGs. 1C1 to 1C4 illustrate primary cultured astrocytes not induced to express a GFAP, and FIGs. 1D1 to 1D4 illustrate primary cultured astrocytes induced to express a GFAP.

### DESCRIPTION OF THE EMBODIMENTS

### 1. Astrocyte nerve-supporting function marker

### 1-1. Outline

A first aspect of the present invention is an astrocyte nerve-supporting function marker. The marker according to the present invention consists of a GFAP. According to the present invention, on the basis of the expression of this marker, an astrocyte having a nerve-supporting function can be identified in astrocytes cultured *in vitro.*

### 1-2. Definitions of terms

The following terms used herein are defined below.

An "astrocyte" is one of the gliacytes present in the central nervous system, and is a cell present in a largest number in the brain of a mammal. As described above, an astrocyte has diverse functions such as the uptake of excessive neurotransmitters and ions, the regulation of the concentration of the extracellular ions around synapses, the control of the blood-brain barrier, energy supply to a neuron, and metabolism.

As used herein, an "astrocyte population" refers to a cell population including a plurality of astrocytes.

A "neuron" is a cell as a basic unit constituting a nervous system. An astrocyte is one of the gliacytes, and has various functions such as the uptake of a neurotransmitter, the maintenance of the ionic environment around synapses, the control of the blood-brain barrier, energy supply to a neuron, and metabolism. A neuron is a cell specific to animals, and has functions specialized in information transmission and information processing.

As used herein, a "marker" refers to a cell marker, in principle. As used herein, a "cell marker" refers to a substance (nucleic acid, polypeptide, or low-molecular-weight compound) that identifies or distinguishes a specific cell, or serves as an index of the existential amount or function of the specific cell.

As used herein, a "function marker" refers to a marker that serves as an index that presents a specific function in a specific cell. In the present invention, the subject is a function marker that presents a nerve-supporting function in an astrocyte in particular, i.e., an astrocyte nerve-supporting function marker the actual substance of which is a GFAP.

As used herein, a "nerve-supporting function" refers to a function that makes neural activities more active in a neuron.

As used herein, the "neural activities" refer to regular and repeated electric excitation caused by an action potential generated by a neuron spontaneously, transmission of the electric signal, and exchange of neurotransmitters between adjacent cells. The neural activities also comprise a state brought about by an action potential generated by a stimulus, the transmission of the electric signal, and exchange of neurotransmitters between adjacent cells. An increase or decrease in the neural activities can be relatively indicated, for example, using the number of Ca²⁺ spikes as an index.

It is known that a "GFAP" (glial fibrillary acidic protein) is a protein constituting a III-type intermediate filament present in the cytoskeleton of an astrocyte, and contributes to the homeostasis of the synaptogenesis and axonal metabolism in the central nervous system.

An "antibody" refers to a polypeptide that contains a framework region (FR) and a complementarity determining region (CDR) both derived from an immunoglobulin, and has a function for specifically binding to an antigen. An "antibody" as singly used herein comprises not only a full-length antibody but also a functional fragment (polypeptide fragment) possessing an antigen-binding capability. The antibody may be a natural antibody or an artificial antibody.

As used herein, a "natural antibody" refers to an antibody produced *in vivo* in a vertebrate, or an antibody having the same amino acid sequence therewith and produced from an antibody-producing cell (for example, a hybridoma) produced artificially. Examples of the natural antibody comprise polyclonal antibodies and monoclonal antibodies. Without limitation, a monoclonal antibody is preferable.

A "polyclonal antibody" refers to a group of a plurality of different immunoglobulins that recognize and bind to different epitopes of the same antigen. A polyclonal antibody can be obtained from a serum of an animal after immunizing the animal against the target molecule as an antigen.

A "monoclonal antibody" refers to a clonal group of single immunoglobulins. The immunoglobulins constituting a monoclonal antibody contain a framework region in common and a complementarity determining region in common, and can recognize and bind to the same epitope of the same antigen. A monoclonal antibody can be obtained from a hybridoma derived from a single cell.

As used herein, an "artificial antibody" is an antibody constructed artificially. Examples comprise an antibody produced by introducing a suitable mutation into the amino acid sequence of the above-described natural antibody; and besides, a single-chain antibody that does not exist in nature in principle, and has resulted from undergoing a structural modification. Specific examples of the latter artificial antibody comprise recombinant antibodies. A "recombinant antibody" refers to a chimeric antibody, humanized antibody, synthetic antibody, or antibody fragment.

A "chimeric antibody" is an antibody produced by combining the amino acid sequences of antibodies derived from different animals, and is an antibody obtained by substituting the variable region (V region) of an antibody with the V region of another antibody. For example, an antibody corresponding to a chimeric antibody is an antibody in which the variable region (V region) is derived from a mouse, and in which the C region is derived from a human, the antibody being obtained by substituting the V region of a mouse monoclonal antibody with the V region of a human antibody.

A "humanized antibody" is a graft antibody obtained by substituting the complementarity determining regions (CDR; CDR1, CDR2, and CDR3) in the variable region (V region) of an antibody of a mammal other than a human, for example, a suitable mouse with the CDRs of a human monoclonal antibody. In a chimeric antibody and a humanized antibody, the V regions of the heavy chain and the light chain are derived, or the complementarity determining regions in the V regions of the heavy chain and the light chain are derived, from an antibody of a non-human animal such as a mouse. The framework regions (FR; FR1, FR2, FR3, and FR4) in the C regions or V regions are derived, and the C regions of the heavy chain and the light chain are derived, from a human antibody. Thus, the immunoreaction with the antibody can be alleviated in the body of a human.

A "synthetic antibody" refers to an antibody synthesized using a chemical method or a recombinant DNA method. Examples comprise a monomer polypeptide molecule obtained by artificially linking one or more VLs and one or more VHs of a specific antibody via a linker peptide having a suitable length and a suitable sequence; and a multimer polypeptide of the monomer polypeptide. Specific examples of such a polypeptide comprise a single-chain Fv (scFv: a single chain fragment of a variable region), diabody, triabody, and tetrabody. These synthetic antibodies are divalent to tetravalent antibody fragments having a dimeric to tetrameric structure respectively based on a single-chain Fv structure. A diabody and a more multiple body can each be a multispecific antibody. A "multispecific antibody" refers to a multivalent antibody, i.e., an antibody having a plurality of antigen-binding sites in one molecule, in which antibody the respective antigen-binding sites bind to different epitopes.

Examples of the "antibody fragment" comprise Fab, F (ab')₂, and Fv.

An "aptamer" is a ligand molecule that binds strongly and specifically to a target molecule based on the steric structure of the molecule. An aptamer has the same effect as an antibody, and can be roughly classified into a nucleic acid aptamer and a peptide aptamer, depending on the kind of the constituent molecule. An aptamer herein may be any of the aptamers. An aptamer, as used singly, refers to a nucleic acid aptamer, unless otherwise specified.

As used herein, a "nucleic acid aptamer" is an aptamer including a nucleic acid, and binds firmly and specifically to a target molecule by virtue of the steric structure formed on the basis of a secondary structure or a tertiary structure which is formed of single-chain nucleic acid molecules via a hydrogen bond. An RNA aptamer including an RNA and a DNA aptamer including a DNA are known. A nucleic acid constituting a nucleic acid aptamer herein is not particularly limited. Examples comprise a DNA aptamer, RNA aptamer, and aptamer including a combination of a DNA and an RNA. The nucleic aptamer usually comprises a naturally occurring nucleic acid (DNA or RNA), but can partly comprise a non-naturally occurring artificial nucleic acid or modified nucleic acid. Without limitation, the base length of an aptamer in the present invention is preferably in the range of from 10 to 100 bases. The length is more preferably in the range of from 15 to 80 bases.

### 1-3. Constitution

An astrocyte nerve-supporting function marker according to the present invention is a GFAP. That is, an astrocyte having a nerve-supporting function for a neuron expresses a GFAP.

In a case where a GFAP is used as a nerve-supporting function marker of an astrocyte, an astrocyte as a target can be a cell cultured *in vitro* or *in vivo,* and is preferably a cell cultured *in vitro.*

A GFAP serving as a nerve-supporting function marker in the present invention is an endogenous protein in an astrocyte, in principle. Some subtypes of GFAPs are known. A GFAP as an astrocyte nerve-supporting function marker according to the present invention may be of any of the subtypes.

The expression level of a GFAP correlates with the strength of the nerve-supporting function. Generally, an astrocyte more highly expressing a GFAP has a stronger nerve-supporting function. It is considered that coculturing a neuron and an astrocyte makes the neural activities more active in accordance with the expression level of a GFAP, and thus, the astrocyte brings about a nerve-supporting function for a neuron.

### 2. Method for identifying astrocyte population having an enhanced nerve-supporting function 2-1. Outline

A second aspect of the present invention is a method for identifying an astrocyte population having an enhanced nerve-supporting function. The method according to the present invention comprises using an astrocyte nerve-supporting function marker in the first aspect to identify whether an astrocyte population has an enhanced nerve-supporting function. According to the method of the present invention, an astrocyte population having an enhanced nerve-supporting function can be readily identified in an astrocyte population cultured *in vitro.*

### 2-2. Method

The identification method according to the present aspect comprises a detection step and an identification step as essential steps, and a culture step as an optional step. The steps are specifically described below.

### 2-2-1. Culture step

The "culture step" is a step of culturing an astrocyte population *in vitro.* The method for culturing an astrocyte population is not particularly limited, and can be a method known in the art. Reference may be made, for example, to a method described by Wu *et al.* (Wu J, Glia, 2010, 58: 315-328). Without limitation, the culture is preferably a primary culture.

The astrocyte can be prepared using a method known in the art. For example, a cerebral cortex is obtained from the whole brain of a mouse or a rat, and then, the cerebral cortex is decomposed in a serum-free medium containing trypsin in the final concentrations, and then dispersed through pipetting to be formed into single cells. Then, the resulting cell dispersion liquid undergoes a permeation culture in a medium supplemented with serum, thus making it possible to obtain astrocytes as adherent cells. Alternatively, an astrocyte can be obtained from a pluripotent stem cell such as an iPS cell or an ES cell through differentiation induction. For a method for inducing an iPS cell to differentiate into an astrocyte, reference can be made, for example, to a known technology described by Kondo (Kondo T, et al., Acta Neuropathol Commun., 2016, 4 (1): 69).

The prepared astrocyte can be seeded in a medium, and then cultured, for example, at 37°C under 5%CO₂. The medium can be a highly versatile basic medium to be used to culture various kinds of cells derived from mammals. Specific examples comprise an Eagle MEM (Eagle Minimum Essential Medium), DMEM (Dulbecco's Modified Eagle Medium), Ham's F10 (Ham's Nutrient Mixture F10) medium, Ham's F12 (Ham's Nutrient Mixture F12) medium, M199 medium, High Performance Medium 199, RPMI-1640 (Roswell Park Memorial Institute-1640) medium, and DMEM/F12 (Dulbecco's Modified Eagle Medium/Ham's Nutrient Mixture F12) medium. In the case of a DMEM/F12 medium, the mixing ratio is not particularly limited. It is preferable that a DMEM and an F12 are mixed in the range of from 6:4 to 4:6 as the ratio of weight concentration of the components. Alternatively, a medium commercially available from a life science manufacturer such as Thermo Fisher Scientific Inc. or FUJIFILM Wako Pure Chemical Industries Corporation can be utilized.

The culture period is desirably at least a time taken until a neuron itself starts the neural activities. After being seeded, neurons are not enabled to perform the neural activities until the neuron elongate neurites, completing the communication between the neurons. Without limitation, the culture is performed for a long period of time, usually 2 weeks to 8 weeks, preferably 5 weeks to 7 weeks. During the culture period, the neurons can be cultured in accordance with a long-term cell culture method known in the art, examples of which the medium is regularly exchanged.

Before the culture with a neuron is started, at least the induction and/or the enhancement of the expression of a GFAP in an astrocyte can be performed. A method for the induction and/or the enhancement of the expression of a GFAP is not limited. Examples comprise a method for imposing stress on an astrocyte, such as a culture at 38°C for 4 hours. A GFAP can be expressed when differentiation from an iPS cell is induced, or can be expressed in an astrocyte after the differentiation induction and in a primary culture.

### 2-2-2. Detection step

The "detection step" is a step of detecting a GFAP expressed intracellularly in an astrocyte population cultured *in vitro.* In the present step, a GFAP is desirably detected without breaking an astrocyte, i.e., with the cellular state retained.

A method for detecting a GFAP is not limited, and the GFAP can be detected usually using a binding factor for a GFAP. Examples comprise an immunological detection method and an aptamer detection method.

### (1) Immunological detection method

The "immunological detection method" is a method in which as a target molecule is an antigen, the target molecule is detected using an antibody that specifically binds to the target molecule to form an immune complex with the target molecule. In the present invention, a GFAP corresponds to the target molecule, and a GFAP expressed in an astrocyte is detected and quantitated using an anti-GFAP antibody.

An anti-GFAP antibody to be used in the present step can be produced using a method known in the art, and using a GFAP as an antigen, or can be produced utilizing an antibody commercially available from life science manufacturers. For example, an anti-GFAP antibody available from Abcam plc, EnCor Biotechnology Inc., or Takara Bio Inc. can be utilized.

Examples of the immunological detection method comprise an enzyme immunoassay, fluoroimmunoassay, luminescent immunoassay, surface plasmon resonance assay (SPR method), quartz crystal microbalance (QCM) assay, radioimmunoassay (RIA), immunonephelometric assay, latex agglutination immunoassay, latex nephelometric assay, particle agglutination assay, gold colloid assay, capillary electrophoresis assay, Western blotting assay, immune cell staining assay, and immunohistochemical analysis (immunostaining) assay. In the present invention, it is desirable that, while an astrocyte cultured *in vitro* is in the cellular state, a GFAP expressed in the astrocyte is detected. Thus, an immune cell staining assay is suitable, without limitation.

The "immune cell staining assay" is a method for detecting an antigen in a cell, using an antibody. According to the method, a cell containing an antigen fluorescently stained through being bound to an antibody can be observed directly under a microscope, and besides that a cell fluorescently stained is quantitated or isolated using a cell sorter (in flow cytometry). The fluorescent labeling antibody can be an anti-GFAP antibody that is a primary antibody, or can be a secondary antibody for an anti-GFAP antibody. A fluorescent substance to be used for fluorescent labeling can be a fluorescent dye known in the art. Examples comprise fluorescein, FITC, rhodamine, Texas red, Cy3, Cy5, Cy7, FAM, HEX, VIC, JOE, Rox, TET, Bodipy 493, NBD, and TAMRA.

A specific method for immunological detection can be in accordance with a method known in the art.

### (2) Aptamer detection method

The "aptamer detection method" is a method in which a target molecule is detected using an aptamer that binds strongly and specifically to the target molecule. In the present invention, a GFAP corresponds to the target molecule, and a GFAP expressed in an astrocyte is detected using an anti-GFAP aptamer that specifically binds to the target molecule. In the present invention, it is desirable that, while an astrocyte cultured *in vitro* is left as it is, a GFAP expressed in the astrocyte is detected. Without limitation, a cell staining assay performed using an aptamer is suitable in the same manner as the immunological detection method. In a cell staining assay to be performed using an aptamer, a basic procedure can be the same as in an immune cell staining assay except that the binding molecule to be used for detection is an anti-GFAP aptamer. For example, an anti-GFAP aptamer to be used can be fluorescently labeled with a fluorescent dye known in the art. A secondary antibody that specifically recognizes this anti-GFAP aptamer can be fluorescently labeled.

An anti-GFAP aptamer can be produced using a method known in the art with a GFAP as a target molecule. For example, an RNA aptamer can be produced by sorting in a test tube, using a SELEX (systematic evolution of ligands by exponential enrichment) method. The SELEX method is a known method, and a more specific method can be performed, for example, in accordance with Pan (Proc. Natl. Acad. Sci. 1995, 92: 11509-11513, U.S.A.).

To simply detect an astrocyte having an enhanced nerve-supporting function, the above-described anti-GFAP antibody or anti-GFAP aptamer can be used as a constituent of the kit for detecting an astrocyte having an enhanced nerve-supporting function.

### (3) Quantitation method

In this step, an astrocyte expressing a GFAP can be detected and also quantitated.

The quantitation method is not limited, and an astrocyte can be quantitated using the fluorescence intensity of a fluorescent dye in the above-described antibody or aptamer used to detect a GFAP. For example, measuring, using a luminometer, the fluorescence intensity in an astrocyte population fluorescently stained using an antibody or an aptamer makes it possible to quantitate an astrocyte expressing a GFAP.

In a case where an astrocyte has an enhanced nerve-supporting function, i.e., an astrocyte expressing a GFAP is isolated singly from an astrocyte population, an astrocyte emitting fluorescence can be sorted out, for example, by flow cytometry.

### 2-2-3. Identification step

The "identification step" is a step of determining, on the basis of the positive rate of an astrocyte population expressing a GFAP in the astrocyte population, whether the astrocyte population is an astrocyte population having a nerve-supporting function or an enhanced nerve-supporting function.

In the present step, the "positive rate" refers to the ratio of cells expressing a GFAP in the cells to an astrocyte population including a predetermined number of cells. The positive rate can be an absolute value calculated from the number of cells expressing a GFAP in the cells with respect to the number of all the cells of an astrocyte population. The positive rate can be a relative value calculated as a ratio of fluorescence intensity of an astrocyte population as a target of measurement, on the basis of the fluorescence intensity per astrocyte obtained in the measurement step, assuming that the fluorescence intensity of an astrocyte population all the cells of which are GFAP-positive is 100%.

Whether the nerve-supporting function of an astrocyte population is increased can be determined on the basis of the positive rate. For example, in a case where the positive rate of an astrocyte population is 20% or more, 30% or more, 40% or more, 50% or more, 60% or more, 70% or more, 80% or more, or 90% or more, an increase in the nerve-supporting function of the astrocyte population can be determined.

### 3. Method for producing coculture

### 3-1. Outline

A third aspect of the present invention is a method for producing a coculture of neurons and an astrocyte population having an enhanced nerve-supporting function. The method according to the present invention comprises mixing neurons and an astrocyte population identified as an astrocyte population having an enhanced nerve-supporting function (an increased-nerve-supporting-function astrocyte population), using the identification method described in the second aspect. According to the present invention, it is possible to obtain a cultured product containing neurons made more active the neural activities.

### 3-2. Method

The production method according to the present aspect comprises a mixing step and a coculture step as basic steps, and comprises an identification step as an optional step. The steps are specifically described below.

### 3-2-1. Mixing step

The "mixing step" is a step of mixing, for the purpose of coculture, neurons and an astrocyte population having a nerve-supporting function or an enhanced nerve-supporting function. Whether an astrocyte population is an astrocyte population having a nerve-supporting function or an enhanced nerve-supporting function can be determined using the identification method in the second aspect. An astrocyte population in the present aspect is typically an astrocyte population identified by the above-described identification method. A neuron to be mixed with an astrocyte population is not particularly limited, and is typically a neuron present in the central nervous system. Examples comprise an excitatory neuron and an inhibitory neuron.

3-2-2. Coculture step

The "coculture step" is a step of culturing the cell population mixed in the mixing step, i.e., an astrocyte population having an enhanced nerve-supporting function and a cell population containing a neuron. The culture method is not particularly limited, and the basic procedure can be performed in accordance with the method described in the culture step in the second aspect. For example, a cell population can be seeded in a medium, and then cultured at 37°C under 5% CO₂. A medium that is optimal differs depending on the cell population to be cultured, and thus, can be suitably selected in accordance with an astrocyte and a neuron that are to be cultured. For example, adding any kind of adjunct to a basic medium described in the culture step in the second aspect makes it possible to select a medium prepared optimally to culture a neuron. For example, a medium for culture of a neuron is commercially available from a manufacturer, and such a medium can be utilized. Other conditions, such as a culture time, can be suitably selected from the conditions described in the culture step in the second aspect.

### 3-2-3. Identification step

The "identification step" is a step of determining whether an astrocyte population is an astrocyte population having a nerve-supporting function or an enhanced nerve-supporting function. The present step is typically performed before the mixing step. The details of the present step can be performed basically in accordance with the method described in the identification step in the second aspect.

### Examples

### <Example 1: Verification (1) of GFAP being astrocyte nerve-supporting function marker>

### (Purpose)

The purpose is to verify that a GFAP serves as a nerve-supporting function marker of an astrocyte.

Whether an astrocyte has a nerve-supporting function and whether the astrocyte is an enhanced nerve-supporting function are detected as the number of the Ca²⁺ spikes in neurons when the neurons are cocultured with the astrocyte. Neurons cocultured with astrocytes having an enhanced nerve-supporting function result in a larger number of Ca²⁺ spikes.

### (Method)

### (1) Induction of differentiation of iPS cell (iPSC) into astrocyte

Differentiation of an iPSC into an astrocyte was induced until day 17, using a differentiation-inducing reagent Quick-Glia^{™} Astrocyte SeV Kit (Elixirgen Scientific, Inc.) in accordance with the attached protocol.

### (2) Induction of GFAP expression in astrocyte

As neurons, human-iPSC-derived excitor neuron Quick-Neuron^{™} Excitatory-Human iPSC-derived neurons (Elixirgen Scientific, Inc.) were used. On day 17 after the start of the differentiation induction, the cells were collected, again seeded in a 6-well plate at 3.7 × 10³ cells/mm², and cultured until day 42. The culture conditions such as a medium were in accordance with the attached protocol.

### (3) Coculture of neurons and astrocytes

A commercially available human-iPSC-derived excitor neuron described in (1) above and a human-iPSC-derived astrocyte obtained through differentiation induction in (1) above or a primary cultured astrocyte (ScienCell Research Laboratories, Inc.) were seeded in mixture in a 384-well plate in such a manner that the number of cells per well was 1 ×10⁴ for neurons and 2.5 ×10³ for astrocytes. The resulting mixture was cocultured at 37°C under 5% CO₂ for 6 weeks. A medium used was Neurobasal Plus (Thermo Fisher Scientific Inc.) supplemented with B-27 Plus Supplement (Thermo Fisher Scientific Inc.), GlutaMAX^{™} Supplement (Thermo Fisher Scientific Inc.), 200 µM ascorbic acid (Fujifilm Wako Pure Chemical Corporation), and 10% Neuron Culture medium (Fujifilm Wako Pure Chemical Corporation).

### (4) Immune cell staining

Immune cell staining was performed, in which the cells at 4% were treated with PFA at room temperature for 15 minutes, then fixed, and blocked with 3% BSA at room temperature for 1 hour. The primary antibodies used were an anti-GFAP antibody (DAKO #GA524; Agilent Technologies, Inc.) not diluted, an anti-S100β antibody (#ab227914; Abcam plc) diluted 1/250-fold, and an anti-ALDH1L1 antibody (#ab190298; Abcam plc) diluted 1/500-fold. Each antibody was stained at 4°C overnight. As a secondary antibody, Goat anti-Mouse IgG (H+L) Cross-Adsorbed Secondary Antibody (Alexa Fluor^{™} 488; Thermo Fisher Scientific Inc.) diluted 1/2000-fold was stained at room temperature for 1 hour. Hoechst 33342 (Thermo Fisher Scientific Inc.) was used for nuclear staining.

### (5) Measurement of Ca²⁺ spikes

To the neurons used, a 5 µM calcium indicator Cal-520^{™} AM (AAT Bioquest, Inc.) was added. The resulting mixture was incubated for 1 hour, and then detected with FDSS µCELL (Hamamatsu Photonics K.K.).

### (Results)

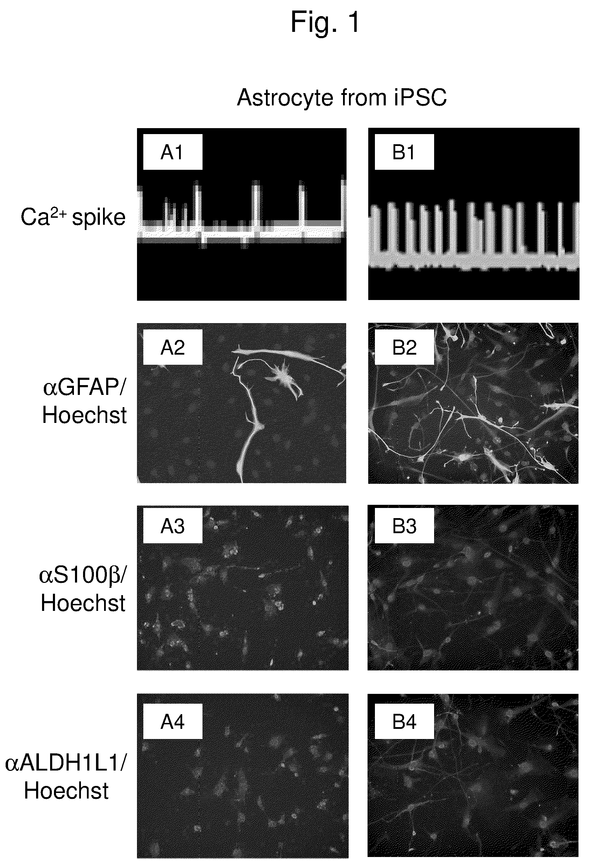

The results are illustrated in FIG. 1. FIGs. 1A1 and 1B1 illustrate the number of Ca²⁺ spikes detected in 20 minutes in neurons cocultured with an astrocyte population for 7 weeks. FIGs. 1A2 and 1B2 are fluorescence staining images obtained using an anti-GFAP antibody for an iPSC-derived astrocyte population used for coculture in FIGs. 1A1 and 1B1 respectively, and using a Hoechst. FIGs. 1A3 and 1B3 are fluorescence staining images obtained using an anti-S100β antibody for an iPSC-derived astrocyte population used for coculture in FIGs. 1A1 and 1B1 respectively, and using a Hoechst. FIGs. 1A4 and 1B4 are fluorescence staining images obtained using an anti-ALDH1L1 antibody for an iPSC-derived astrocyte population used for coculture in FIGs. 1A1 and 1B1 respectively, and using a Hoechst. S100β and ALDH1L1 are markers for identifying an astrocyte.

The number of Ca²⁺ spikes in FIG. 1A1 was 5.8 (CV = 0.71), the GFAP positive rate in FIG. 1A2 was less than 10%, the S100β positive rate in FIG. 1A3 was 75%, and the ALDH1L1 positive rate in FIG. 1A4 was 80%. The number of Ca²⁺ spikes in FIG. 1B1 was 13.3 (CV = 0.24), the GFAP positive rate in FIG. 1B2 was more than 80%, the S100β positive rate in FIG. 1B3 was 75%, and the ALDH1L1 positive rate in FIG. 1B4 was 80%.

The astrocyte population in FIGs. 1A and the astrocyte population in FIGs. 1B did not present a large difference in the positive rates of S100β and ALDH1L1. The GFAP positive rate was singly high for the astrocyte populations in FIGs. 1B. The number of Ca²⁺ spikes was twice or more as high for the astrocyte population in FIGs. 1B as for the astrocyte populations in FIGs. 1A.

The above-described results have revealed that the nerve-supporting function is enhanced in the astrocyte populations having a high GFAP positive rate, thus suggesting that a GFAP can serve as a nerve-supporting function marker in an astrocyte.

### <Example 2: Verification (2) of GFAP being astrocyte nerve-supporting function marker>

### (Purpose)

In Example 1, an astrocyte prepared by differentiation induction from an iPS cell was used. In this Example, the purpose is to verify that a GFAP can serve as a nerve-supporting function marker even in a primary cultured cell of an astrocyte.

### (Method)

The basic experimental procedure was in accordance with the method described in Example 1. A commercially available primary cultured astrocyte (ScienCell Research Laboratories, Inc.) was purchased, and 1 × 10⁶ cells were thawed and seeded in a dish. As a medium, an astrocyte medium (ScienCell Research Laboratories, Inc.) was used, and the cells were cultured at 37°C under 5% CO₂ for 1 week. The cells were collected in accordance with the attached protocol, and freeze-preserved. In each experiment, the cells freeze-preserved were thawed, mixed with neurons, and cocultured.

### (Results)

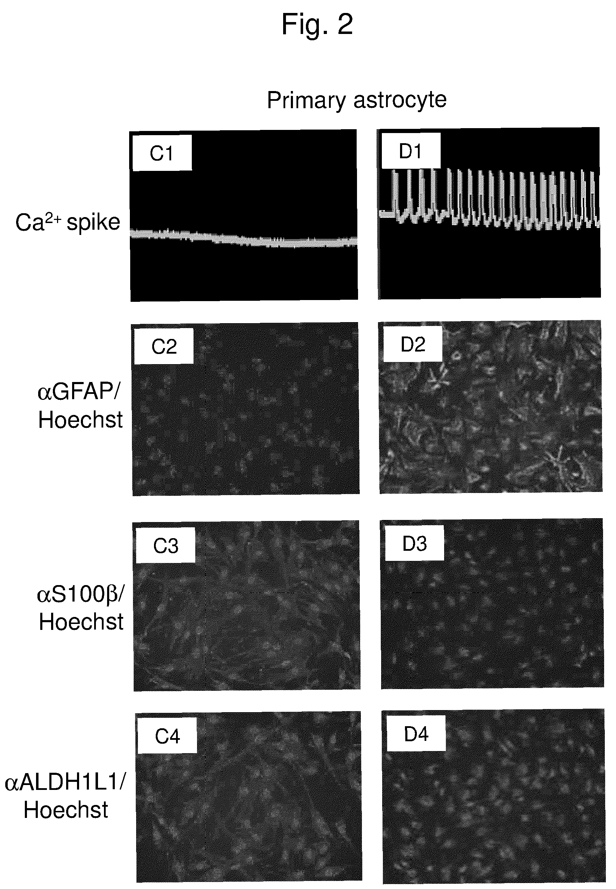

The results are illustrated in FIG. 2. FIGs. 2C1 and 2D1 illustrate the number of Ca²⁺ spikes detected in 20 minutes in neurons cocultured with an astrocyte population for 7 weeks. FIGs. 2C2 and 2D2 are fluorescence staining images obtained using an anti-GFAP antibody for an iPSC-derived astrocyte population used for coculture in FIGs. 2C1 and 2D1 respectively, and using a Hoechst. FIGs. 2C3 and 2D3 are fluorescence staining images obtained using an anti-S100β antibody for an iPSC-derived astrocyte population used for coculture in FIGs. 2C1 and 2D1 respectively, and using a Hoechst. FIGs. 2C4 and 2D4 are fluorescence staining images obtained using an anti-ALDH1L1 antibody for an iPSC-derived astrocyte population used for coculture in FIGs. 2C1 and 2D1 respectively, and using a Hoechst. S100β and ALDH1L1 are markers for identifying an astrocyte.

The number of Ca²⁺ spikes in FIG. 2C1 was 0, the GFAP positive rate in FIG. 2C2 was 0%, the S100β positive rate in FIG. 2C3 was 85%, and the ALDH1L1 positive rate in FIG. 2C4 was 85%. The number of Ca²⁺ spikes in FIG. 2D1 was 20.5 (CV = 0.28), the GFAP positive rate in FIG. 2D2 was more than 90%, the S100β positive rate in FIG. 2D3 was 90%, and the ALDH1L1 positive rate in FIG. 2D4 was 90%.

Also with the primary cultured astrocytes, comparison between the astrocyte populations in FIGs. C and the astrocyte populations in FIGs. D has revealed that, between the astrocyte populations not having a large difference in the S100β and the ALDH1L1 positive rate, the astrocyte population having a higher GFAP positive rate exhibited a larger number of Ca²⁺ spikes. This result agrees with the result in Example 1, and demonstrates that a GFAP serves as a nerve-supporting function marker in an astrocyte, whatever cell the astrocyte can be derived from.

### Citation List

### Non-Patent Documents

Non-Patent Document 1: Hasan U. & Singh S.K., Astrocytes Method and Protocols, 2019: 3-18.
Non-Patent Document 2: Chipman P.H., et al., eLife, 2021, https://doi.org/10.7554/eLife.70818
Non-Patent Document 3: Ahtiainen A., et al., Int J Mol Sci, 2021, 22(23): 12770.
Non-Patent Document 4: Jurga AM, et al., Biomolecules, 2021, 11(9), 1361.
Non-Patent Document 5: Lung D., et al., J Clin Invest., 2019, 129(10): 4408-4418.

## Claims

1. A method comprising:
detecting a glial fibrillary acidic protein (GFAP) in an astrocyte population cultured *in vitro,* and
determining whether the astrocyte population is an astrocyte population having a nerve-supporting function or an enhanced nerve-supporting function on the basis of the positive rate of the astrocyte population having the GFAP expressed in the astrocyte population.

2. An astrocyte nerve-supporting function marker consisting of a GFAP.

3. A kit for detecting an astrocyte having a nerve-supporting function or an enhanced nerve-supporting function, comprising at least one of an anti-GFAP antibody and/or an anti-GFAP aptamer.

4. A method for producing a cell culture, comprising:
mixing step of mixing a neuron and an astrocyte population identified as having a nerve-supporting function or an enhanced nerve-supporting function; and
coculture step of culturing the astrocyte population after the mixing step.
